# FASCICULE DE BREVET EUROPEEN

(11) **EP 0 729 930 B1**
(45) Date de publication et mention de la délivrance du brevet: **17.12.2003**
(21) Numéro de dépôt: 96400375.0
(22) Date de dépôt: 23.02.1996
(51) Int. Cl.: C07B 39/00, C07C 319/20, C07C 323/03, C07C 323/08, C07C 323/52, C07D 231/44, C07D 317/46, C07C 315/02, C07C 317/44, C07C 313/04

(54) **Procédé de synthèse de composés hydrocarbonés fluorés sur un carbone d'une chaîne alcoyle**
Verfahren zur Herstellung von auf einem Kohlenstoffatom in einer Alkylkette fluorierten Kohlenwasserstoff
Process for the synthesis of hydrocarbons fluorinated on a carbon of an alkyl chain

(30) Priorité: 28.02.1995 FR 9502290
(43) Date de publication de la demande: 04.09.1996
(73) Titulaire: RHODIA CHIMIE, 92408 Courbevoie Cédex (FR)
(72) Inventeur: Janin, Robert, 69960 Corbas (FR); Saint-Jalmes, Laurent, 69330 Meyzieu (FR)
(74) Mandataire: Ricalens, François

(56) Documents cités:
- EP-A- 0 423 009
- EP-A- 0 590 459
- US-A- 5 196 599
- JOURNAL OF FLUORINE CHEMISTRY, vol. 59, no. 3, Décembre 1992, LAUSANNE, CH, pages 417-422, XP002004227 K. MORIMOTO, ET AL.: "Reaction of quinoxalin-2-ones with difluorocarbene"
- TETRAHEDRON LETTERS, vol. 22, no. 21, 1981, OXFORD, GB, pages 1997-2000, XP002004228 M. SUDA, ET AL.: "Preparation of bromodifluoromethyl sulphide and its conversion to trifluoromethyl sulphide"
- JOURNAL OF ORGANIC CHEMISTRY, vol. 44, no. 4, 16 Février 1979, WASHINGTON, DC, US, pages 563-569, XP002004229 J.F. HARRIS, JR.: "Free-radical reactions of fluoroalkanesulphenyl halides. 3. Reactions of fluoroalkane- and chlorofluoroalkanesulphenyl chlorides with hydrocarbons"
- CHEMICAL ABSTRACTS, vol. 105, no. 19, 10 Novembre 1986 Columbus, Ohio, US; abstract no. 171888m, A. HAAS, ET AL.: "Catalytic addition of trihalogenomethanesulphenyl chlorides to olefinc compounds" page 691; XP002004232 & JOURNAL OF FLUORINE CHEMISTRY, 1985, 30(2), 203-210:
- JOURNAL OF THE CHEMICAL SOCIETY, PERKIN TRANSACTIONS 1, no. 24, Décembre 1992, LETCHWORTH, GB, pages 3371-3375, XP002004230 J.-L. CLAVEL, ET AL.: "Reactions of bromotrifluoromethane and related halides. Part 12. Transformation of disulphides into perfluoroalkyl sulphides in the presence of sulphoxylate anion radical precursors"
- JOURNAL OF THE AMERICAN CHEMICAL SOCIETY, vol. 95, no. 10, 16 Mai 1973, WASHINGTON, DC, US, pages 3412-3413, XP002004231 J.B. HENDRICKSON, ET AL.: "Triflamides (CF3SO2N<). A survey"

## Description

La présente invention a pour objet un procédé de synthèse de composés hydrocarbonés fluorés sur un carbone d'une chaîne alcoyle par échange entre un halogène de rang plus élevé et le fluor au moyen d'un réactif contenant du fluor au moins partiellement sous forme de sels complexes. Elle concerne plus particulièrement un procédé permettant l'obtention de dérivés fluorés sur un atome de carbone portant des groupes électroattracteurs par effet inductif.

Les composés fluorés sont en général difficiles d'accès. La réactivité du fluor est telle qu'il est difficile voire impossible d'obtenir directement les dérivés fluorés.

Une des techniques les plus employées pour fabriquer le dérivé fluoré consiste à faire réagir un dérivé halogéné, en général chloré, pour échanger l'halogène avec un fluor minéral, en général un fluorure de métal alcalin, en général de poids atomique élevé.

En général, le fluorure utilisé est le fluorure de potassium qui constitue un compromis économique satisfaisant.

Dans ces conditions, de nombreux procédés tels que par exemple ceux décrits dans le certificat d'addition N° 2 353 516 et dans l'article Chem. Ind. (1978) - 56 ont été décrits et mis en oeuvre industriellement pour obtenir des fluorures d'*aryle, aryles* sur lesquels sont greffés des groupements électro-attracteurs.

Sauf dans les cas où le substrat est particulièrement adapté à ce type de synthèse, cette technique présente des inconvénients dont les principaux sont ceux que l'on va analyser ci-après.

La réaction nécessite des réactifs comme les fluorures de métal-alcalin tels que le fluorure de potassium qui sont rendus relativement chers par les spécifications auxquels ils doivent répondre pour être adaptés à ce type de synthèse ; ils doivent être très purs, secs et sous une forme physique adaptée.

Ainsi dans la demande de brevet européen N°423 009, la demanderesse a démontré qu'il était possible d'utiliser dans quelques cas très spécifiques du fluorure de potassium pour réaliser un échange chlore/fluor sur un carbone aliphatique en présence de fluorure acide de potassium.

En outre cette réaction ne marche pas pour toute une classe de produits notamment ceux portant sur le carbone halogénophore (c'est à dire le carbone portant le ou les halogènes destinés à être échangés avec le fluor).

Il est aussi utilisé des réactifs tels que l'acide fluorhydrique liquide ou dilué par des solvants aprotiques dipolaires. Un exemple d'une telle utilisation est donné par la demande de brevet européen N°590 459 où des benzodioxols sont soumis à l'action d'acide fluorhydrique pour réaliser l'échange. Bien que le substrat s'y prête, pour éviter la formation de goudron de manière trop abondante, on est obligé de travailler à des températures très basses, c'est-à-dire à des températures inférieures à - 10°C. Toutefois l'acide fluorhydrique est un réactif trop puissant et conduit souvent à des réactions de polymérisation indésirées ou à des goudrons.

Dans ce cas, et notamment dans le cas où l'on désire des dérivés fluorés sur un carbone de type alcoyle (y compris aralcoyle) appauvri en électron par la présence de groupes de type électroattracteur, l'homme de métier se trouve devant une alternative dont les termes ne sont guère encourageants; ou bien l'on choisit des conditions très dures et l'on obtient surtout des goudrons, ou bien on l'on se place dans des conditions réactionnelles douces et l'on retrouve, dans le meilleur des cas le substrat inchangé. Enfin il convient de signaler que certains auteurs ont proposer de réaliser des échanges en utilisant comme réactif des sels de l'acide fluorhydrique en présence d'éléments lourds sous forme d'oxydes ou de fluorures. Parmi les éléments utilisés, il convient de citer l'antimoine et les métaux lourds tel que l'argent ou le vif-argent (mercure).

Un autre problème réside dans la sélectivité de la réaction : lorsque il y a plusieurs halogènes à échanger sur le même carbone, il est souvent difficile de n'en échanger qu'une partie.

C'est pourquoi un des buts de la présente invention est de fournir un procédé qui soit susceptible de réaliser l'échange entre d'une part les halogènes lourds tels que le chlore et d'autre part le fluor en améliorant significativement la spécificité de la réaction. Un autre but de la présente invention est de fournir un procédé qui soit susceptible de réaliser l'échange entre d'une part les halogènes lourds tels que le chlore et d'autre part le fluor en utilisant des conditions réactionnelles particulièrement douces

Un autre but de la présente invention est de fournir un procédé qui permette d'utiliser une source de fluorure dont la morphologie soit moins critique.

Un autre but de la présente invention est de fournir un procédé qui permette de n'échanger qu'un atome d'halogène sur deux ou sur trois possibles.

Un autre but de la présente invention est de fournir un procédé qui permette de n'échanger que deux atomes d'halogène sur trois possibles.

Un autre but de la présente invention est de fournir un procédé qui permette de n'échanger les molécules ou les atomes que dans la mesure où cela permet d'obtenir des atomes de carbone qui ne soient porteurs que d'un atome de fluor concomitamment avec un ou deux autres halogènes distincts du fluor.

Un autre but de la présente invention est de fournir un procédé qui permette de n'échanger les molécules ou les atomes que dans la mesure où cela permet d'obtenir des atomes de carbone qui ne soient porteurs que de deux atomes de fluor concomitamment avec un autre halogène distinct du fluor.

Un autre but de la présente invention est de fournir un procédé qui permette d'éviter l'usage de forte quantité de métaux réputés coûteux ou toxique tel que le mercure et/ou l'argent.

Un autre but de la présente invention est de fournir un procédé qui permette de réduire les quantités de métaux réputés coûteux ou toxique tel que le mercure et/ou l'argent, de manière que le rapport molaire entre le métal et le substrat dont les atomes d'halogène sont à échanger, se situe à une valeur au plus égale à 0,5, avantageusement à 0,2, de préférence à 0,1.

Un autre but de la présente invention est de fournir un procédé qui permette d'éviter complètement l'utilisation de métaux réputés coûteux ou toxique tel que le mercure et/ou l'argent, de manière à n'ajouter au mélange réactionnel aucun des éléments cités ci-dessus en d'autres termes que les concentrations en chacun des dits métaux ne dépassent pas les valeurs de 10⁻³ M; avantageusement 10⁻⁴ M, de préférence de 10⁻⁵ M.

Le document publié par Katsuschi Morimoto, et al., dans le Journal of Fluorine Chemistry, 59 (1992) 417-422, Reaction of Quinoxaline 2 one quinoxalin-2-with difluorocarbene indique que, en présence de sel ou d'oxyde de mercure, il est possible dans l'isopropanol de réaliser l'échange du brome avec un complexe HF pyridine mais il ne s'agit que du dernier échange et il n 'y a aucun enseignement qu'il puisse y avoir une sélectivité.

Par ailleurs, dans le brevet américain US P N°5 196 599, l'échange chlore/fluor est décrit pour une variété très spécifique de composés nitrofluoroformals difluoroformals mais cet échange est décrit comme complexe et aucune caractéristique de sélectivité n'est décrite.

Ces buts et d'autres qui apparaîtront par la suite sont atteints au moyen d'un procédé utile pour la synthèse de dérivés fluorés qui comporte une étape d'échange entre le fluor et un halogène plus lourd que le fluor, dans laquelle un substrat comportant au moins un carbone halogénophore d'hybridation sp³ porteur d'au moins deux halogènes ci-après désignés par X et X', dont l'un au moins est un halogène plus lourd que le fluor, lequel carbone halogénophore est relié à un chalcogène au moins, et soumis à l'action d'un réactif comportant au moins un composé défini choisi parmi ceux constitués par l'association d'une base de Bronstedt avec un nombre défini n d'acides fluorhydriques, n étant au moins égal à 3 et au plus à 20, avantageusement, de préférence à 10, et par le fait que ledit substrat correspond à la formule :

*R-CXX'-Y(O)*_{*r*}*-R*_{*5*}

- avec R choisi parmi les restes hydrocarbonés, les halogènes, les électro-attracteurs et les hydrocarbyles chalcogényles ;
- avec X représentant le le chlore ;
- avec X' représentant le chlore ;
- avec Y choisi parmi les chalcogènes avantageusement de rang atomique supérieur à l'oxygène, notamment quand R est différent des hydrocarbyles chalcogényles et avec la condition que lorsque Y est oxygène, R est égal à 0 ;
- où r représente 0 ou un entier choisi parmi 1 ou 2 et est avantageusement inférieur à 2 ;
- R₅ représente un radical :
   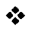 aryle éventuellement substitué, en particulier hétéroaryle ;
   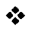 alcoyle dont le carbone d'hybridation sp³ constitue un ensemble moins éléctroattracteur qu'un dichlorophényle;
et par le fait que chaque radical R et R₅ comporte au plus 30, de préférence au plus 15 atomes de carbone et/ou d'azote, le nombre total de carbones des molécules substrats ne dépassant pas 50, avantageusement 30.

En effet au cours de l'étude qui a mené à la présente invention, il a été montré que certains atomes de carbone portant des groupes électroattracteurs par effet inductif, sous la condition qu'au moins un des groupes électroattracteurs fût chalcogène, étaient susceptibles de réagir avec un réactif du type ci dessus.

La température de réaction varie du point de fusion du mélange réactionnel à son point de décomposition ou d'ébullition, en général de 0°C à 150°C, avantageusement de 20 à 100°C.

On travaille en général à la pression atmosphérique, mais il est possible de travailler à des pressions pouvant atteindre 20 10⁵ Pascals.

Parmi les bases préférées, on peut citer celles qui sont des dérivés trivalents hydrocarbonés des éléments de la colonne VB, avantageusement de période d'un rang au moins égal à la deuxième et en général inférieure à la sixième, de la classification périodique des éléments (supplément au Bulletin de la Société Chimique de France Janvier 1966 N°1). Outre ceux qui sont détaillés par la suite on peut donner comme exemples de tels composés les dérivés trivalents, qui lorsqu'ils sont trisubstitués sont en fait des pnictines, pnictines qui font l'objet d'une description plus détaillée ci-après.

Parmi lesdits dérivés hydrocarbonés des éléments de la colonne V les préférés sont ceux qui dérivent des pnictures d'hydrogène par substitution totale ou partielle de l'hydrogène par des restes hydrocarbonés qui peuvent être reliés à l'atome de la colonne V B par une double (comme dans les imines) ou une triple liaison (comme dans les nitriles).

Toutefois les dérivés hydrocarbonés des éléments de la colonne V dérivent avantageusement des pnictures d'hydrogène par substitution totale ou partielle de l'hydrogène par des restes hydrocarbonés monovalents avantageusement par des alcoyles [dans la présente description **ALCO-*yle*** est pris dans son sens étymologique de reste hydrocarboné d'un **ALCO-*ol*** après ignorance de la fonction alcool (ou ol)]; ces composés alcoylés seront, par analogie avec le terme de pnicture, désignés dans la présente description, sous le terme de pnictines.

Ainsi dans le cas de l'azote la substitution du nitrure d'hydrogène (ammoniac) donne les amines, dans le cas du phosphore la substitution du phosphure d'hydrogène donne les phosphines, dans le cas de l'arsenic la substitution de l'arséniure d'hydrogène donne les arsines et dans le cas de l'antimoine la substitution de l'antimoniure (ou stibiure) d'hydrogène donne les stibines. Ils sont avantageusement choisis parmi les dérivés hydrocarbonés du phosphore tels que les phosphines.

Par ailleurs, plus la base est faible et plus elle est molle, meilleur et plus complet est l'échange. Ainsi les amines primaires, secondaires et de préférence tertiaires conduisent à des réactifs contenant peu de groupes HF (au plus 5, en général moins) et moins puissants que les bases de type hétérocycle aromatique dont le ou au moins l'un des hétéroatomes est choisi dans la colonne V.

Ces composés formé d'une base et d'un nombre discret d'unité HF seront désignées ci-après sous le terme de complexe(s) "HF Base" ou "Base HF".

La présente invention ne vise pas les échanges avec les fluorures métalliques (notamment alcalins tel que KF, CsF ..) cela peut s'exprimer par le fait que la quantité [(exprimée en équivalent) de cation(s) (alcalins, ammoniums)], doit être au moins égale à une fois (avantageusement au moins à 4/3 de fois, de préférence à 2 fois environ) celle de l'hydrogène sous forme de proton libre, d'acide halohydrique dégagé, ou de complexes "base-HF" y compris "F-(HF)".

On peut donner la règle empirique suivante: si les bases forment des composés définis de plus de 5 HF par fonction basique (le paradigme des réactifs forts est le composé défini pyridine, 10 HF), alors c'est un réactif puissant susceptible d'échanger deux halogènes lourds sur le même carbone dans des conditions très douces et même trois dans des conditions un peu plus dures (température et pression). Sinon il s'agit d'un réactif plus sélectif qui n'échange, en général, que pour donner un seul fluor sur un carbone dans des conditions douces et deux fluors sur le carbone dit halogénophore dans des conditions plus sévères. Cette invention est surtout intéressante pour remplacer des chlores par des fluors.

Ainsi, les réactions d'échange sont essentiellement successives (en effet chaque atome de fluor supplémentaire sur le carbone halogénophore ralentit l'échange des atomes d'halogène plus lourds que le fluor avec ce dernier), ce qui permet de réaliser un échange sélectif ou complet, en jouant sur les conditions opératoires et sur te choix des réactifs. Comme il est en général possible de trouver des conditions où la réaction d'échange s'arrête avant que la totalité des halogènes plus lourds que le fluor ne soit remplacé par ce dernier, il s'ensuit qu'une sélectivité à deux volets est possible. D'une part il possible de n'échanger qu'un nombre limité des halogène plus lourd que le fluor et d'autre part il est possible de traiter un mélange déjà partiellement fluorés et de ne toucher de manière significative que les molécules n'ayant pas atteint le nombre désiré d'atome de fluor.

En général la facilité d'échange d'un atome d'halogène plus lourd que le fluor avec ce dernier, croît avec son nombre atomique.

Bien évidemment on peut jouer sur la stoechiométrie et son excès pour limiter le nombre des atomes d'halogène échangés par molécule.

Il peut y avoir plusieurs atomes de carbone halogénophore par molécule. Il est préférable que deux atomes halogénophores n'interfèrent pas l'un avec l'autre. On donnera ci-après une typologie des atomes de carbone, voire des molécules, les plus aptes à échanger leurs halogènes lourds avec le fluor sous l'action des réactifs ci-dessus. Chaque caractéristique ci-après rend plus vif l'intérêt de l'invention pour lesdits carbones.

Ainsi il est particulièrement intéressant que l'éventuelle liaison résiduelle du carbone halogénophore soit avantageusement une liaison avec un groupe choisi. parmi les groupes électroattracteurs par effet inductif. Ledit groupe choisi parmi les groupes électroattracteurs est avantageusement un halogène.

Pour obtenir une bonne réactivité, il est préférable que la somme des nombres atomiques dudit ou desdits chalcogènes(s) soit au moins égal à 10. En d'autres termes, s'il n'y a qu'un seul chalcogène il est préférable qu'il soit choisi parmi les chalcogènes plus lourds que l'oxygène. l'invention est particulièrement intéressante lorsque l'un des chalcogènes au moins est un soufre.

L'invention est particulièrement utile quand ledit carbone halogénophore porte au moins deux halogènes de nombre atomique supérieur à celui du fluor.

Ainsi que l'on l'exposera plus tard, l'invention est particulièrement intéressante lorsque ledit carbone halogénophore est trihalogénométhyle, c'est-à-dire qu'il porte trois halogènes choisis avantageusement parmi le chlore et le fluor.

Si l'on s'intéresse au(x)dit(s) chalcogène(s), il convient de noter qu'il est de préférence divalent (nombre d'oxydation = -2) quand il est seul . Et que lorsqu'ils sont deux, au moins un d'entre eux est divalent, l'autre pouvant être simplement électroattracteur, en raison par exemple de liaison(s) de type donneur-accepteur entre ledit chalcogène [avec, bien entendu car cela n'aurait pas de sens chimique, la condition qu'il ne soit pas oxygène] et l'oxygène (par exemple, sulfone ou sulfoxyde).

Ainsi, pour paraphraser ce qui précède, et quitte à être superfétatoire, on peut indiquer que les substrats peuvent être des molécules de formule I :

R-CXX'-Y(O)ᵣ-R₅ (I) (I)

avec R choisi parmi reste hydrocarbonés, les halogènes, les électroattracteurs et les hydrocarbylchalcogényles tels que alcoxyles, aryloxyles et leur correspondants sulfuré, sélénié et telluré ;
avec X représentant le chlore et avec X' représentant le chlore ;
avec Y choisi parmi les chalcogènes, avantageusement de rang atomique supérieur à l'oxygène notamment lorsque R est différent de hydrocarbylchalcogényle et avec la condition que lorsque Y est oxygène, r est égal à zéro ;
r représente zéro ou un entier choisi parmi un ou deux et est avantageusement inférieur à 2 ;
avec R₅ défini comme précédemment.

Lorsque le radical R n'apporte pas de chalcogène divalent (c'est-à-dire dont les deux doublets sont disponibles), il est souhaitable que r soit inférieur à deux, de préférence soit égal à zéro.

Lorsque R₅ est électroattracteur, surtout par effet mésomère, il convient de noter que l'échange est plus difficile, surtout pour le troisième atome de fluor sur le même carbone.

Aussi pour un échange complet, Il est souhaitable que ledit chalcogène soit lié par sa seconde liaison à un atome électrodonneur par effet inductif ou mésomère. Ledit atome électrodonneur peut être un autre chalcogène (lequel est donneur par effet mesomère), avantageusement de rang atomique supérieur à celui de l'oxygène.

Ledit atome électrodonneur peut également être un carbone appartenant à un radical alcoyle ou à un radical arylique riche en électron. Dans ce cas ledit alcoyle est avantageusement un radical aralcoyle, de préférence choisi parmi les radicaux benzyles et ledit radical arylique riche en électron est avantageusement un radical hétérocyclique à cinq chaînons ou homocyclique à six chaînons.

Aussi pour un échange conduisant à trois atomes de fluor sur le même carbone halogénophore, il est préférable que R₅ soit alcoyle, c'est-à-dire que sa liaison d'ancrage soit portée par un carbone d'hybridation sp³ ; avantageusement ledit carbone d'hybridation sp³ porte des substituants qui globalement constituent un ensemble non ou faiblement attracteur (c'est-à-dire moins attracteur qu'un dichlorophényle) ; de préférence ledit carbone d'hybridation sp³ porte au moins un, avantageusement deux hydrogènes.

Toutefois, en général, même ainsi, cette valeur R = H n'est pas préférée.

Avantageusement R₅ peut notamment être :
- aryle éventuellement substitué, en particulier hétéroaryle ;
- alcoyle et notamment :
   ⇒ -CR'R"-Ar ;
      → avec les R' et R" semblables ou différents choisis parmi l'hydrogène, les aryles et les alcoyles légers (c'est-à-dire de 1 à 4 atomes de carbone) et de préférence, l'un ou les deux sont avantageusement hydrogène ;
      → Ar est un composé présentant au moins une double liaison et dont le carbone porteur de la liaison ouverte est un carbone SP¹ et de préférence Sp². Ar est avantageusement un aryle léger, ayant de préférence au plus 10 atomes de carbone et étant avantageusement homocyclique ;
   ⇒ -CR'R"-CR₁R₂-GEA ;
      → où les radicaux R' et R" sont définis ci dessus ;
      → GEA représente un Groupe ElectroAttracteur, un groupe stabilisant une double liaison ou un groupe partant ;
      → R₁ et R₂-semblables ou différents sont choisis parmi l'hydrogène, les halogènes, des restes hydrocarbonés notamment les alcoyles, les ynes, les alcènes, les aryles; l'un ou les deux sont avantageusement hydrogène.

Chaque radical R et R₅ comporte habituellement au plus 30 (dont au plus 20 atomes de carbone), avantageusement 20 (dont au plus 15 atomes de carbone), de préférence 15 atomes de carbone et/ou d'azote (dont au plus 12 atomes de carbone). Le nombre total de carbones des molécules substrats dépasse rarement 50, et est avantageusement d'au plus 30.

Lorsque R₅ est aryle mention particulière peut être faite des composés où :
→ R est alcoyle léger [éventuellement substitué, et notamment halogéné (y compris perhalogéné et notamment perfluoré)], halogène, aryle ou Ar'O- et Ar'S- avec Ar' représentant un aryle léger (c'est-à-dire de 10 atomes de carbone au plus) ;
→ R₅ représente un cycle phényle éventuellement substitué, un hétérocycle éventuellement substitué, avantageusement à cinq chaînons, de préférence à deux hétéroatomes (il est souhaitable d'avoir deux atomes d'azote) ; ainsi par exemple le radical -Y(O)ᵣ-R5 répond avantageusement à la formule (X): 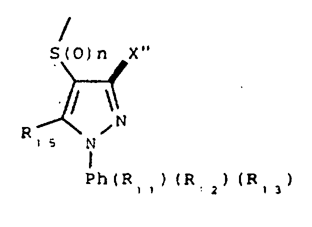
   où n peut prendre les mêmes valeurs que r à savoir 0 ; 1 ou 2 ;
   où R₁₁ et R₁₂ semblables ou différents, avantageusement en position ortho, représentent un hydrogène ou un halogène ;
   où R₁₃, avantageusement en position para, représente un halogène, un groupe alcoyle éventuellement substitué par un ou plusieurs halogènes (y compris un groupe choisi parmi les perfluoroalcoyles), un groupe alcoyloxyle éventuellement substitué par un ou plusieurs halogènes (y compris un groupe choisi parmi les perfluoroalcoyloxyles), ou un radical SF₅.
   Où X" représente une fonction nitrile ou un halogène
   où R₁₅ peut être un groupe amino, éventuellement mono ou disubstitué par des radicaux (semblables ou différents, en cas de disubstitution) choisi parmi tes alcoyles éventuellement substitués par un ou plusieurs halogènes (y compris les perfluoroalcoyles), les acyles éventuellement substitués par un ou plusieurs halogènes (y compris les perfluoroacyles), et les alcoyloxycarbonyles.
      Les alcoyles, les alcoyloxyles, et les acyles sont de préférence légers c'est-à-dire qu'ils comportent au plus quatre atomes de carbone.
      Il est à noter que lorsque R₁₃,représente un groupe alcoyloxyle éventuellement substitué par plusieurs halogènes et que l'un au moins desdits halogènes est de rang atomique supérieur à celui du fluor, il y a deux centres carbonés d'échange possibles.

Les restes R₅ et R peuvent constituer un seul et même radical mais divalent. Par exemples, ils peuvent constituer un radical aryle, les points d'attache étant portés soit par deux carbones d'un même noyau et en position vicinale l'un de l'autre; soit par deux carbones en bêta l'un de l'autre, n'appartenant pas au même noyau, mais dont les deux noyaux sont jointifs et condensés (e.g. Cas de deux positions alpha du naphtalène ou équivalentes) ; soit par deux carbones en γ l'un de l'autre appartenant des noyaux séparés par un tiers noyau à la manière du phénanthrène.

A titre de paradigme de telles formules on peut donner les exemples suivants :
cas des carbones vicinaux d'un même noyau : 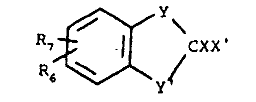 avec Y' pouvant être soit une simple liaison, soit un chalcogène (avec les mêmes préférences que Y), soit un méthylène éventuellement mono ou disubstitué par des halogènes, soit un groupe divalent -Y"-CΞΞ'- ou -CΞΞ'-Y"- ; avec Y" pouvant prendre les mêmes valeurs que Y, et, Ξ et Ξ' les mêmes valeurs que X et X' respectivement (Ξ et Ξ' peuvent être simultanément fluor).
   R₆ et R₇ pouvant être indépendamment un hydrogène, un halogène, groupe nitro, un nitrile, un groupe carboné, avantageusement d'au plus 5 atomes de carbone, un groupe alcoyle éventuellement substitué par un ou plusieurs halogènes (y compris un groupe choisi parmi les perfluoroalcoyles), un groupe alcoyloxyle éventuellement substitué par un ou plusieurs halogènes (y compris un groupe choisi parmi les perfluoroalcoyloxyles), ou un radical SF₅.

Et notamment : 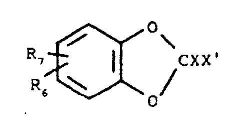 cas des carbones de deux noyaux distincts avec R₈ choisi parmi les mêmes valeurs que R₆ ou R₇:

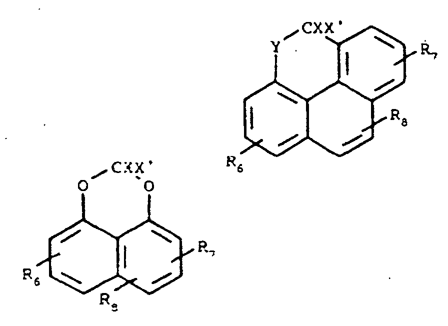

Selon une version particulièrement avantageuse de l'invention, on peut réaliser des coupures (ou des lyses) pour former des composés particulièrement utiles pour la synthèse organique et pour la synthèse d'acides chalcogénophores (et dont le chalcogène présente un rang au moins égal à celui du soufre). L'oxydation des composés selon l'invention peut être mené par des peroxydes et notamment par ceux des l'hydrogène (eau oxygénée et divers hydroperoxydes [par exemple d'acyle, d'alcoyle] dans des conditions en elles-mêmes connues, ou par les halogènes et notamment le chlore. Pour cette variante il est souvent intéressant que ledit chalcogène soit du soufre et qu'il comporte une étape ultérieure d'oxydation dudit atome de soufre. ladite étape d'oxydation est avantageusement réalisée pour obtenir ledit atome de soufre sous forme d'une sulfone. Ladite oxydation peut être aussi réalisée pour obtenir ledit atome de soufre sous la forme d'un sulfoxyde, d'un sulfénate (sel ou ester) ou sous une forme oxydée présentant le même état d'oxydation que celui d'un sulfoxyde (Par exemple halogénure de sulfinyle [-S(O)-Cl]), ou que celui d'un un sulfénate (Par exemple halogénure de sulfényle [-S-Cl]).
Ultérieurement cette variante peut comporter une étape ultérieure d'hydrolyse avantageusement en milieu alcalin pour donner un sel l'acide sulfinique ou sulfonique correspondant.
Ce type de réaction peut conduire selon les conditions soit à des sulfényles soit à des sulfoxydes, soit enfin comme cela à été déjà indiqué à des lyses conduisant à des acides sulfonique ou sulfinique ou à leurs équivalents lorsque le chalcogène est sélénium ou tellure en lieu et place de soufre.

En outre, de manière surprenante, il a été montré que les sulfénates dont le carbone vicinal du soufre est perfluoré, lors de leur oxydation par des halogènes (avantageusement le chlore), si on utilise seulement la stoechiométrie ou un léger excès stoechiométrique, (quantité d'halogène allant de 0,5 a 1,5 QS, avantageusement de 8 à 1,3, de préférence de 0,9 à 1,2QS) conduisent à l'halogénure de sulfinyle.

La dite halogénation est menée en soumettant ledit sulfénate, avantageusement dilué dans un solvant peu polaire (c'est-à-dire incapable de dissoudre plus de 5 % en masse d'eau), essentiellement anhydre (c'est-à-dire que dans le milieu réactionnel l'eau est présente en quantité d'au plus 1/3 en mole du substrat, avantageusement d'au plus 1/5, de préférence d'au plus 1/10) et peu sensible au chlore, à l'action du chlore au moins en quantité sensiblement stoechiométrique, à une température au plus égale à 100°C, avantageusement entre 0°C et 50°C.

La réaction en cause est la suivante :

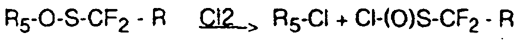

Cette réaction présente un intérêt particulier pour les R d'au plus 10 atomes de carbone. elle marche d'autant mieux que le carbocation R₅⁺ est plus stable ; ainsi pour cette dernière réaction, R₅ est avantageusement benzylique, allylique ou tertioalcoyle. Ainsi parmi les substrats idoines on peut citer ceux de formule II, avec Y soufre ou les chalcogènes supérieurs et r égal à 1 et l'oxygène avantageusement intercalé entre Y et CR'R".

Il convient de rappeler que les halogénures et notamment les chlorures de sulfinyles (de type R-CF₂ -SO-) sont des intermédiaires de synthèse particulièrement importants.

Les molécules particulièrement aptes à cette réaction sont surtouts celle de formule (II) dérivée de la formule I

R-CFX-Y(O)ᵣ-CR'R"-Ar (II)

avec R choisi parmi les halogènes, les électroattracteurs, les hydrocarbyles, tels que alcoyles et les aryles, et les hydrocarbylchalcogényles tels que alcoxyles, aryloxyles et leurs correspondants sulfurés, séléniés et tellurés ;
avec X choisi parmi les halogènes de préférence le chlore et surtout le fluor ;
avec Y choisi parmi les chalcogènes, avantageusement de rang atomique supérieur à l'oxygène et avec la condition que lorsque Y est oxygène, r est égal à zéro r représente zéro ou un entier choisi parmi un ou deux ;
avec les R' et R" semblables ou différents choisis parmi les aryles et les alcoyles légers et de préférence, l'un ou les deux sont hydrogènes.
Ar est un composé présentant au moins une double liaison et dont le carbone porteur de la liaison ouverte est un carbone sp¹ et de préférence sp². Ar est avantageusement un aryle léger, ayant de préférence au plus 10 atomes de carbone et étant avantageusement homocyclique.

Selon une variante avantageuse de la présente invention, II est possible de faire subir au composés de formule 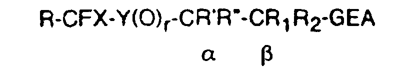 une β élimination,
Formule dans laquelle au moins avantageusement deux, l'un des radicaux R', R", R₁ ou R₂ représente un hydrogène (Il est souhaitable que l'un des radicaux sur chacun des carbones α et β soit hydrogène) ;
GEA représente un Groupe ElectroAttracteur, un groupe stabilisant une double liaison ou un groupe partant (dans le cas ou on veut former par β élimination avec coupure entre le carbone β et GEA, un dérivé de formule -CFX-Y(O)ᵣ-CR'R"=CR₁R₂).

Comme groupe électro attracteur on peut choisir les halogènes, les groupes à fonction carbonyles (tels que amide, ester, cétones, aldéhydes), les groupes dérivés du carbonyle (tels que imines, amidine, oxime, thiocétonique, thioesters, thioamides, thioloesters) les nitriles, les pnictoniums (notamment phosphoniums, ammoniums, voir infra..) le groupe nitro, les orthoesters, les radicaux dont au moins l'atome vicinal de la liaison libre (ou ouverte, c'est-à-dire la liaison qui relie le radical au reste de la molécule considérée) est perhalogéné et notamment perfluoré, ainsi on peut préciser que conviennent les radicaux perfluoroalcoyles tels que le trifluorométhyle, le pentafluoroéthyle, ainsi que les difluoros-1,1 et tétrafluoro-1,1,2,2 alcoyles, tels le difluoro-1,1 éthyle, le difluoro-1,1 et le tétrafluoro-1,1,2,2 propyle.

On peut également citer comme groupes électro-attracteurs les groupes dérivés des chalcogènes oxygénés (tel que les sulfoxydes, les sulfones) ou des éléments de la colonne VB de la classification périodique tels que les oxydes de phosphine et les esters des acides phosphoniques ou phosphiniques ; la liaison libre est avantageusement portée par le métalloïde (chalcogène ou élément de la colonne VB).

Toujours dans le cas des bêta éliminations, comme groupe partant outre les halogènes déjà mentionné ci dessus, on peux citer les pseudohalogènes dans l'acception ci-après. Dans la présente description, est considéré comme pseudohalogène un radical (en général ce radical possède un chalcogène léger (soufre ou de préférence oxygène) par lequel il est relié au reste de la molécule) qui en partant constitue un anion dont l'acide associé présente une acidité (mesurée par la constante de Hammett) au moins égale à celle de l'acide acétique. Parmi les pseudohalogènes typiques on peut citer les radicaux acyloxyles correspondant aux acides perhalogénés en alpha de la fonction acyloxyle, tels le trifluoroacétyloxyle (CF₃ - CO-O-), et surtout les radicaux sulfonyloxyles, surtout ceux dont le carbone porteur du soufre est perfluoré dont le paradigme est le trifluorométhylsulfonyloxyle (CF₃ - SO₂-O-).

Pour la présente invention, on préfère ceux des pseudohalogènes qui en partant présentent une acidité au moins égale à celle des acides sulfoniques, tel le tosylique (paradigme des acides arylsulfoniques) ou le mésylique (paradigme des acides alcoylsulfoniques).

Dans la présente description un pnictonium est une pnictine tertiaire quaternisée par un groupe hydrocarbyle (tel que aryle ou alcoyle y compris aralcoyle).

Lesdites pnictines sont des dérivés trivalents hydrocarbonés des éléments de la colonne VB. Elles dérivent des pnictures d'hydrogène par substitution totale ou partielle de l'hydrogène par des restes hydrocarbonés qui peuvent être relié à l'atome de la colonne V B par une double (comme dans les imines) ou une triple liaison (comme dans les nitriles).

Toutefois les dérivés hydrocarbonés des éléments de la colonne V dérivent avantageusement des pnictures d'hydrogène par substitution totale ou partielle de l'hydrogène par des restes hydrocarbonés monovalent avantageusement par des alcoyles [dans la présente description **ALCO-*yle*** est pris dans son sens étymologique de reste hydrocarboné d'un **ALCO-*ol*** après ignorance de la fonction alcool (ou ol)]; ces composés alcoylés sont, par analogie avec le terme de pnicture, désignés dans la présente description, sous le terme de pnictines.. Ainsi dans le cas de l'azote la substitution du nitrure d'hydrogène (ammoniac) donne les amines, dans le cas du phosphore la substitution du phosphure d'hydrogène donne les phosphines, dans le cas de l'arsenic la substitution de l'arséniure d'hydrogène donne les arsines et dans le cas de l'antimoine la substitution de l'antimoniure (ou stibiure) d'hydrogène donne les stibines. Ils sont avantageusement choisis parmi les dérivés hydrocarbonés du phosphore tels que les phosphines.

En général il est désiré que la β élimination se fasse avec coupure entre Y(O)ᵣ et CR'R" auquel cas, Il faut que l'un au moins des R₁ et R₂ soit hydrogène et auquel cas il est préférable que GEA représente un groupe électroattracteur ou un groupe stabilisant une double liaison. Comme groupe stabilisant une double liaison, il convient de citer tout groupe possédant une liaison susceptible d'être conjuguée avec un éventuelle double liaison entre les carbones α et β; outre les radicaux électroattracteurs présentant une double liaison déjà énuméré ci dessus, on peut citer comme de tel groupe les ynes, les alcènes, les aryles.

Il est également souhaitable que GEA soit suffisamment électroattracteur pour stabiliser un carbanion en position β; Lorsque GEA est aryle et notamment phényle éventuellement substitué, il est souhaitable que R₁ et/ou R₂ soient eux même choisi parmi les radicaux adapté à GEA et notamment parmi les ynes, les alcènes et les aryles.

Enfin toujours dans le cas d'une β élimination avec coupure entre Y(O)ᵣ et CR'R", il est souhaitable GEA soit un groupe partant médiocre et avantageusement moins bon que R-CFX-Y(O)ᵣ. Il est également avantageusement que r soit au moins égal à 1, de préférence soit égal à 2.

La réaction se fait selon des techniques en soi connues en utilisant des bases fortes dont le Pka de l'acide associé est avantageusement au moins égal à 14. pour aider l'homme de métier à déterminer les conditions à choisir suivant les cas rencontrés, on trouvera ci après des règles empirique utilisable dans la majorité des situations rencontrées.

Rappelons que l'expression "hydrocarbylchalcogényle" est un radical de structure R₆-Y"- où R₆- est un radical hydrocarboné, c'est-à-dire comportant au moins de l'hydrogène et du carbone et dont l'atome porteur de la liaison (ici avec Y") est un carbone, et où Y" est un chalcogène (oxygène, soufre, sélénium, tellure). R₆ est avantageusement un alcoyle [éventuellement substitué, et notamment halogéné (y compris perhalogéné et notamment perfluoré)] ou un aryle éventuellement substitué.

Les références des radicaux se rapportent à la formule (I) ;

R-CXX'-Y(O)ᵣ-R₅ (I)

Par électrodonneur ou faible électroattracteur, il convient de comprendre aussi attracteur ou moins attracteur qu'un dichlorophényle (définition qui convient aussi pour aryle non électroattracteur ou pour un radical arylique riche en électron). Par différence, on déduit la définition dans le tableau ci après "d'électroattracteur" ou de "significativement électroattracteur"
Le paradigme des réactifs faibles est le composé défini triéthylamine, 3 HF
Le paradigme des réactifs forts est le composé défini pyridine, 10 HF
conditions douces : θ = point de fusion à 50°C au plus ;
conditions dures : 50°C à 100°C (ou au point d'ébullition s'il est plus bas à la pression considérée) ;
conditions très dures :θ = 100 à 150°C et le cas échéant pressions supérieures à l'atmosphérique ;

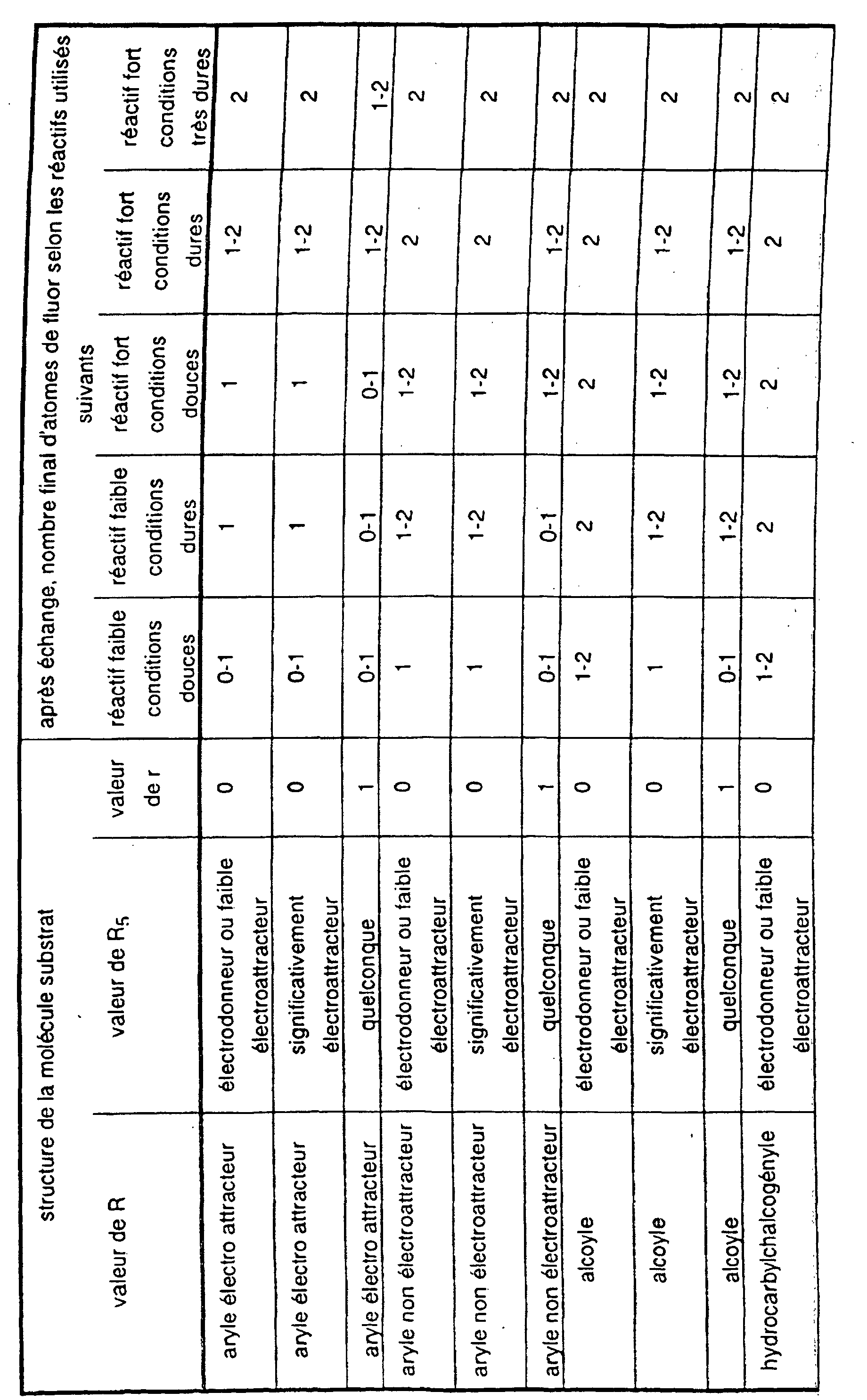

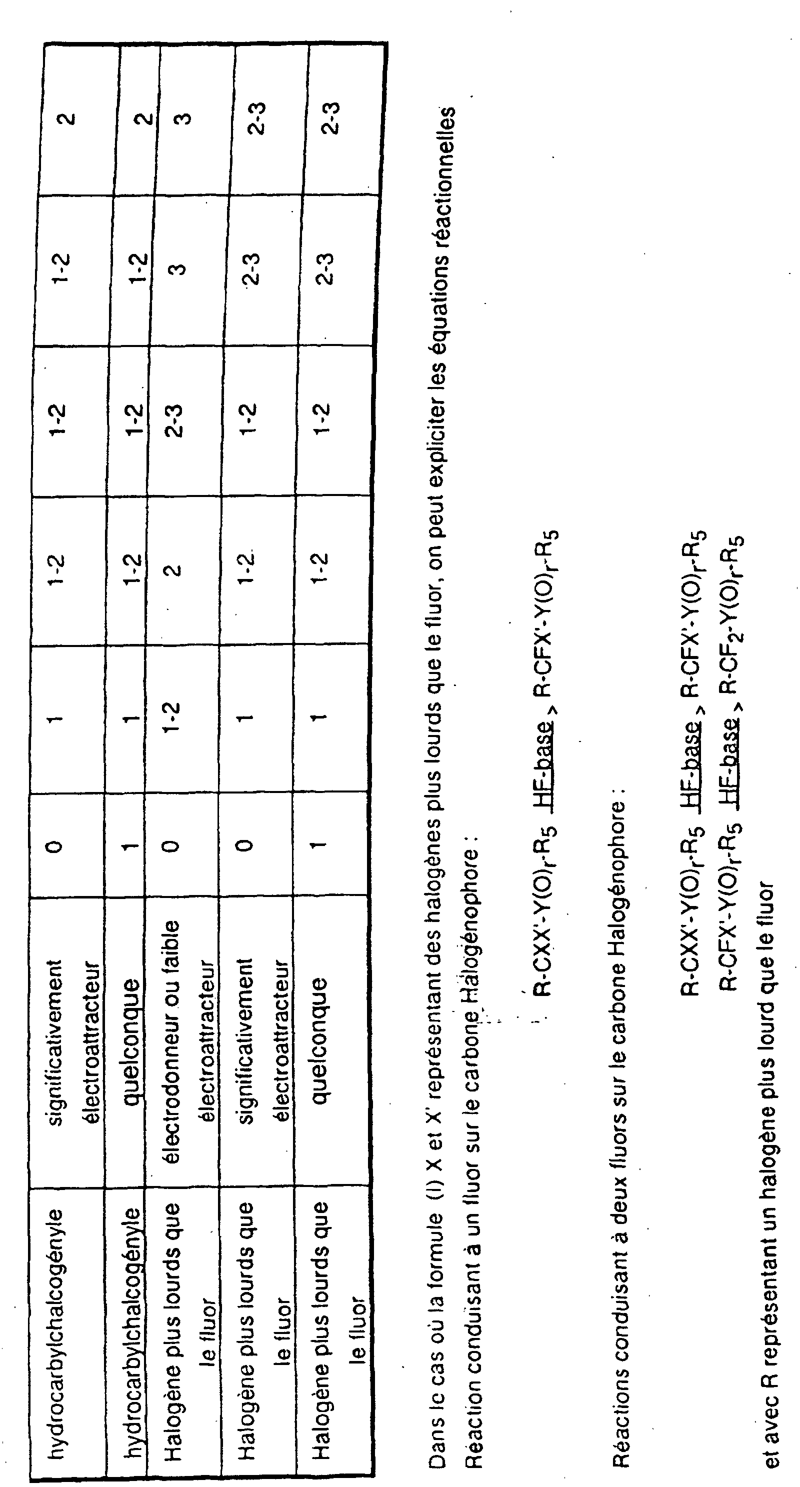

Réactions conduisant à trois fluors sur le carbone Halogénophore :

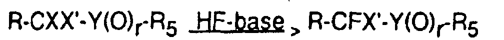

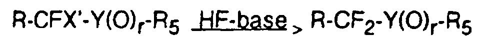

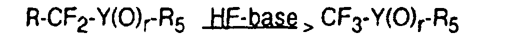

Les exemples non limitatifs suivants illustrent l'invention.

### Exemple N°1. Préparation des Solutions HF - base - Mode opératoire général

Les différents milieux HF - base sont synthétisés comme suit :

A x moles d'une base organique (pyridine, triéthylamine, dioxane, ...) ou minérale (KF, bu₄NF, ...) sous agitation (éventuellement refroidies à -20°C) sont ajoutées Y moles d'acide fluorhydrique anhydre goutte à goutte. Après addition de l'acide fluorhydrique anhydre, le milieu réactionnel est réchauffé à température ambiante et utilisé sans aucun traitement. Le complexe HF - base a donc la structure (HF)_{y} - Baseₓ. Après la réaction, Lorsque le brut de fluoration est traité par une phase organique anhydre non miscible avec le milieu HF-Base considéré, mais apte à dissoudre le produit d'arrivée (Par exemple uniquement du CH₂Cl₂ (sans glace ou eau)) 2 phases sont obtenues: la phase la moins polaire (Par exemple CH₂Cl₂) qui contient le produit obtenu après échange, et la phase la plus polaire "HF - base" qui peut être alors recyclée, après une éventuelle remise au titre initial (en HF) et élimination de l'acide halohydrique dégagé par la réaction (Par exemple par distillation). Ce recyclage est propre au procédé selon la présente invention et est un avantage supplémentaire du procédé.

### Exemple N°2 Echange Cl-F à partir du trichlorométhylthiobenzyle synthèse du substrat

### Equation réactionnelle

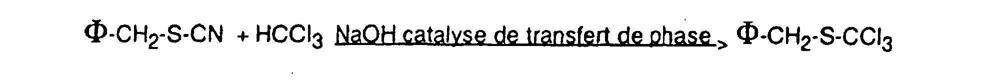

### Mode opératoire utilisé

Le trichorométhylthiobenzyle 1 est obtenu suivant les travaux de M. Makosza (synthesis (1974) 274).

### 2a) Echange de trois chlores

### Equation réactionnelle

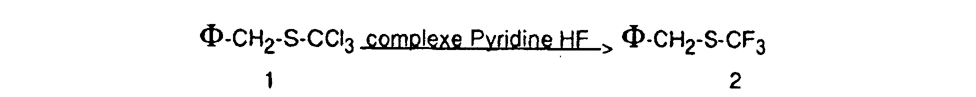

### Mode opératoire utilisé

90,1 g (0,374 mole) du sulfure 1 sont additionnés à 297 g de complexe [HF]₁₁-pyridine 1 (préparé à partir de 77,3 g de pyridine et 219,7 g d'acide fluorhydrique anhydre) refroidi à 0°C.

Le milieu réactionnel est ensuite ramené à température ambiante et agité pendant 18h.

Le brut réactionnel est alors versé sur un mélange de CH₂Cl₂ (5200 ml) et de glace (500g).

La phase organique est lavée 4 fois par 100 ml d'eau, séchée sur sulfate de magnésium. Le solvant (CH₂Cl₂) est évaporé pour fournir 68 g de sulfure 2 (95 % par rapport à 1) qui peut être purifié par distillation (Eb = 77/30 mm Hg)
RMN 19_{F} = 35,9 ppm (référence : TFA - acide trifluoro acétique)

Lorsque le brut de fluoration est versé sur uniquement du CH₂Cl₂ (sans glace ou eau) 2 phases sont obtenues : la phase CH₂Cl₂ qui contient le sulfure 2, la phase HF - pyridine qui peut être recyclée.

### 2b)

### Equation réactionnelle

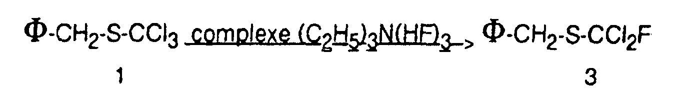

### Mode opératoire utilisé

2 g (8,3.10⁻³ mole) de sulfure 1 sont ajoutés à 20 ml de complexe (HF)₃, Et₃N à température ambiante. Le milieu réactionnel est agité 4 h à 20°C puis est versé dans un mélange de CH₂Cl₂ (100 ml) et de glace 25 g et d'eau 50 ml. La phase organique est lavée 3 fois à l'eau (50 ml) puis séchée sur MgSO₄. L'évaporation du solvant fournit 1,7 g du sulfure 3 (91,5 %).

### 2c)

### Equation réactionnelle

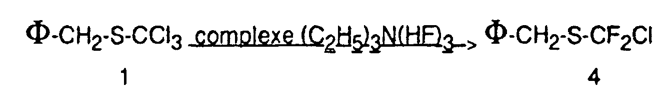

### Mode opératoire utilisé

Les conditions sont les mêmes que dans la réaction précédente, mais le milieu réactionnel est agité 18H à 75°C. Le traitement est le même que dans l'essai précédent et conduit à 1,64 g de sulfure 4 (95 % par rapport à 1).
RMN 19F = 50,8 ppm (référence TFA).

### Exemple N°3 Echange sur le sulfure de Chloro-2 cyclohexyle et de trichlorométhyle

### Equation réactionnelle

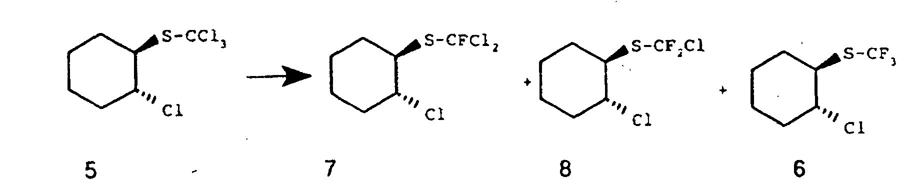

### Mode opératoire utilisé

Le sulfure initial (noté 5 ) (sulfure de Chloro-2 cyclohexyle et de trichlorométhyle) est obtenu par transaddition de CCl₃ SCI sur le cyclohexène.

1 g (3,73. 10⁻³ mole) du sulfure 5 est ajouté sur le complexe [HF]₁₀₋ pyridine 1 (5g) refroidi à 0°C. Le milieu réactionnel est ensuite agité pendant 6 h à température ambiante avant d'être versé sur un mélange de CH₂Cl₂ (50 ml) et d'eau - glace (50 g).

La phase organique est lavée 3 fois par 50 ml d'eau puis séchée sur MgSO₄. Le solvant est évaporé pour fournir 0,73 g de sulfure 6 (90 % par rapport à 5). ¹⁹F = 167 ppm (référence TFA [acide trifluoroacétique])

### 3b) échange partiel

1 g (3,73. 10⁻³ mole) de sulfure 5 est ajouté à température ambiante à 10 ml du complexe [HF]₃ - Et₃N. Le milieu réactionnel est agité 5 h à température ambiante. Le traitement est le même que dans l'essai précédent et fournit 0,85 g de sulfure 7 (91 % par rapport à 5) accompagné du sulfure 8 :

### Exemple N°4 Synthèse d'acide triflinique (trifluorométhanesulfinique)

### Equations réactionnelles

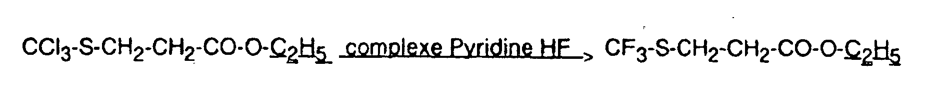

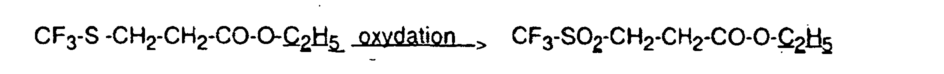

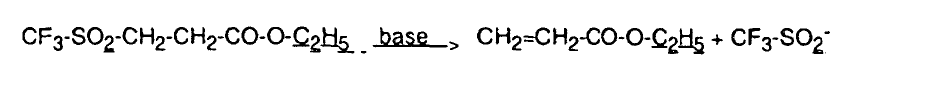

### 4a) Echange sur CCl₃S(CH₂)₂CO₂Et 9

### Mode opératoire utilisé

100 mg de sulfure 9 sont ajoutés à 0°C à 1 ml de complexe [HF]₁₀₋ pyridine 1. Le milieu réactionnel est ensuite agité 24 h à température ambiante puis est versé sur un mélange de CH₂Cl₂ (20 ml) et d'eau/glace (20g).

La phase CH₂Cl₂ est lavée 3 fois par 10 ml d'eau, séchée sur MgSO₄ puis le solvant CH₂Cl₂ est évaporé pour fournir une huile jaune (80 mg) qui contient environ 50 % de sulfure 10.
RMN 19_{F} du sulfure 10 : - 41,93 ppm ( % CFCl₃).

### Exemple N°5

### 5) Echange sur des dérivés du pyrazole

R-CXX'-Y(O)ᵣ-R₅ (I) → R-CFX'-Y(O)ᵣ-R₅ + R-CF₂-Y(O)ᵣ-R₅ + FCF₂-Y(O)ᵣ-R₅

**avec R halogène (ici chlore) ; X =X=Chlore ; Y = S ; r = 0 et**
R₅ = 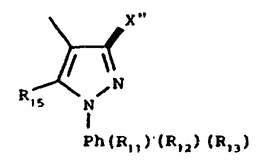 X" = nitrile ; R₁₁ = R₁₂ = ortho Chloro et R₁₃= para trifluorométhyle

### Mode opératoire général

Le pyrazole trichlorométhylé 11 est additionné sur un mélange HFₓ - base_{y} (x, y connus). Le mélange réactionnel est agité à une température et pendant une durée donnée.

Après retour à température ambiante, le bruit réactionnel est versé sur un mélange CH₂Cl₂ / eau/glace. La phase organique CH₂Cl₂ est lavée 3 fois à l'eau, séchée sur MgSO₄, puis le solvant est évaporé.
L'analyse des bruts obtenus est réalisé par HPLC.
Les résultats des principaux essais réalisés sont réunis dans le tableau de la page suivante :

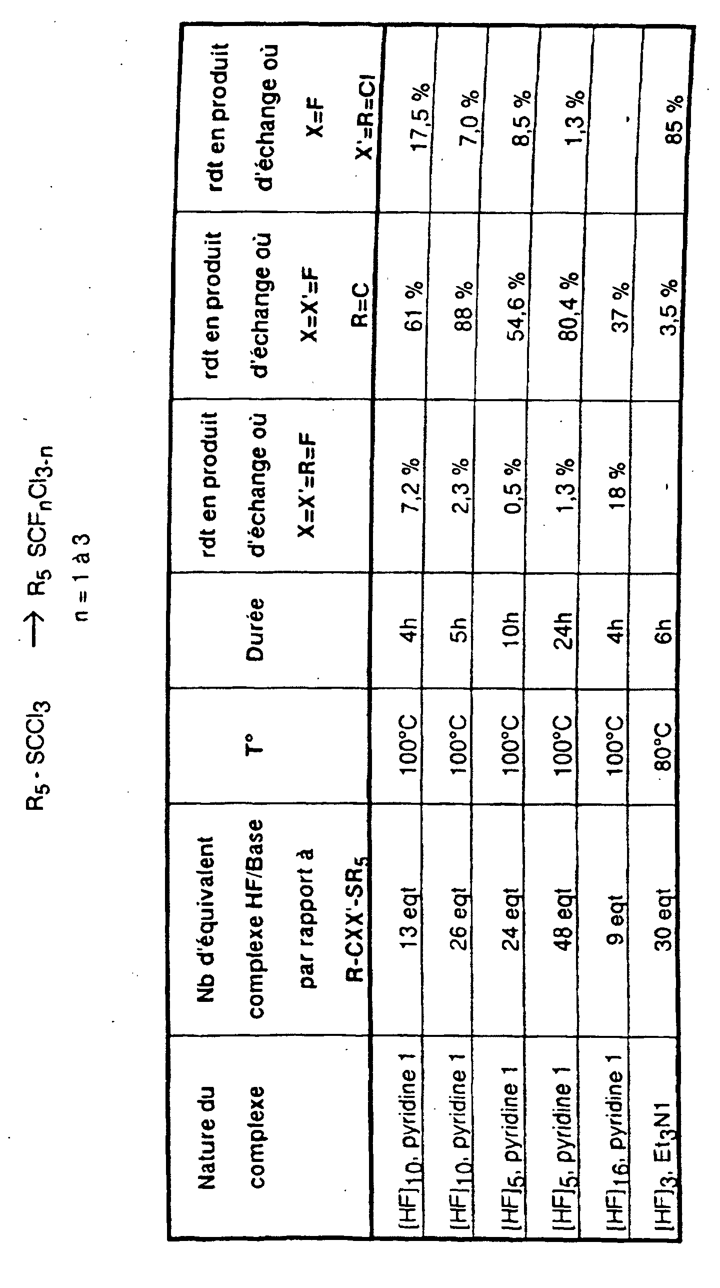

### Exemple N°6) Echange Cl - F sur dichloro MDB.

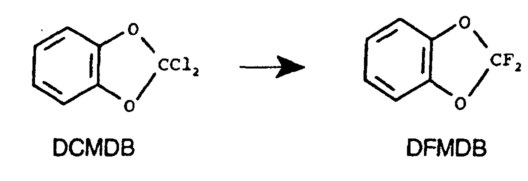

### 6a) Echange avec [HF]₁₀ pyridine 1

La mesure du rendement est semi quantitative (analyse CPV).

191 mg (1 mmole) de DCMDB sont ajoutés à 0°C à 2 ml d'[HF]₁₀, pyridine 1. Le milieu réactionnel est agité 2 h à température ambiante, puis est versé sur un mélange CH₂Cl₂/eau/glace. Après lavage de la phase organique, l'analyse CPV donne
- DFMDB: ≃ 95 % (aire CPV)
- DCMDB: ≃ quantité difficilement évaluable (au plus égale à 1 %)
- Fluoro chloro MDB :: ≃ 1 %

### 6b) Echange avec [HF]₃, Et₃N1

La mesure du rendement est semi quantitative (analyse CPV).
191 mg de DCMDB sont ajoutés à 2 ml d'[HF]₃, Et₃N à 0°C.
Le temps de réaction à température ambiante est de 3 h.
Le traitement est le même que dans l'essai précédent.
L'analyse CPV donne :
- DFMDB: ≃ 90 % (aire CPV)
- DCMDB: ≃ quantité difficilement évaluable (au plus égale à 10 %)

### 6c) Echange DCMDB → DFMDB avec des milieux HF + base "catalytique"

Dans les essais suivants, la quantité de base (organique ou minérale) est beaucoup plus faible que dans les essais précédents. Les complexes HF-base catalytique sont formés de la même manière que les complexes HF - base utilisés précédemment.
Le tableau suivant (page suivante) montre l'influence de quantité catalytique de base (Et₃N et KF) par rapport à un essai "blanc" sans base ajoutée.
Le traitement des essais par HF - base catalytique est le même que ceux des essais avec [HF]₁₀, pyridine 1 et [HF]₃, Et₃N1.

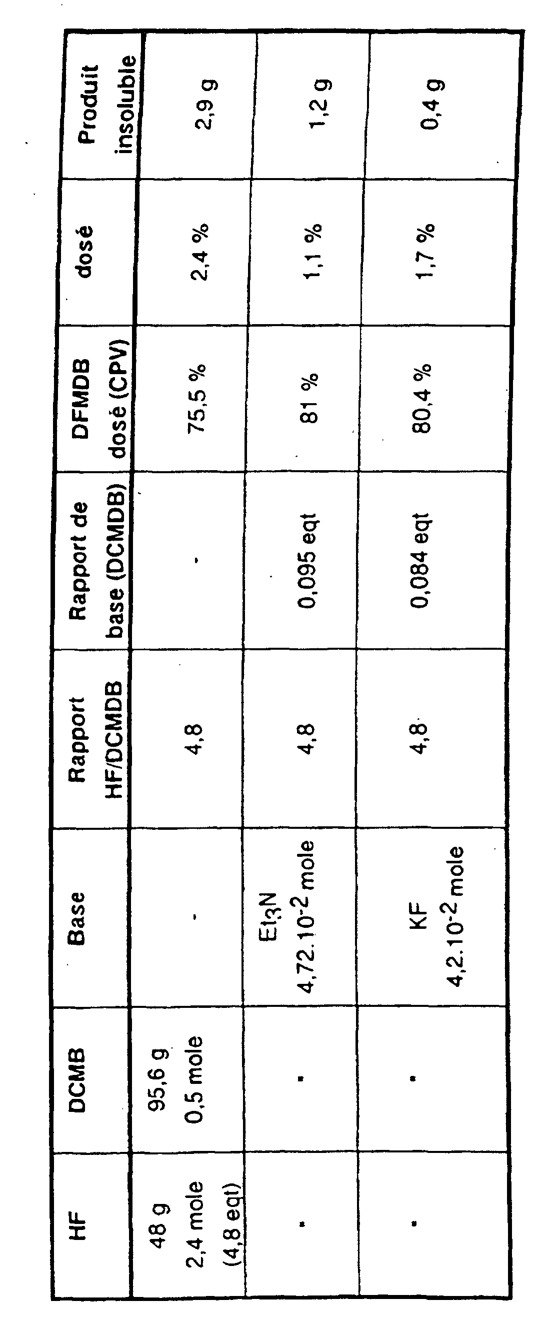

### exemple N°7 Oxydation pour donner un sulfénate

### équation réactionnelle

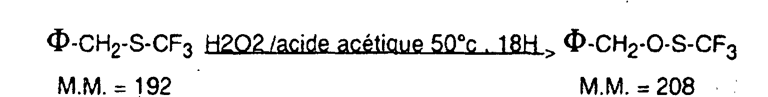

### Mode opératoire

A 192 mg (1eq.) de sulfure de benzyle et de trifluorométhyle dilué dans 1 ml d'acide acétique, on ajoute 0,11 ml (1,1 eq.) d'eau oxygénée à 30 %.
Le milieu est chauffé à 50°C pendant 18 H. Le brut réactionnel est repris dans du dichloro méthane, la phase organique est lavée à l'eau puis séchée sur sulfate de sodium. Après évaporation on obtient un solide blanc identifié comme le sulfénate attendu et en quantité correspondant à un rendement de 90 %.

### Exemple N°8 Oxydation du sulfénate

### équation réactionnelle

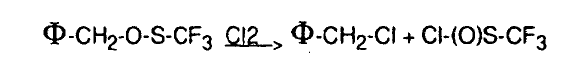

### Mode opératoire

A 208 mg (1eq.) de trifluorométhylsulfénate de benzyle dilué dans 2 ml de chlorure de méthylène (c'est-à-dire du dichlorométhane CH₂Cl₂), on ajoute 71 mg (1,1 eq.) de chlore gazeux. Après 18h à température ambiante. Une analyse par chromatographie en phase vapeur (souvent désignée par ses initiales : CPV) montre que la transformation du substrat est totale ainsi qu'un rendement en chlorure de benzyle et en chlorure de trichlorométhane sulfinyle de respectivement 75 % et 80 %.

### Exemple N° 9

### Synthèse de PhCH₂SCCl₃

Le thiocyanate de benzyle (29.8 g, 0.2 mol), le chloroforme (76 g, 0,6 mol) et le chlorure de triéthyl benzyl ammonium (0.5 g, 0.002 mol) sont agités vigoureusement. On ajoute lentement à cette solution 40 ml de soude aqueuse à 50 % (faible exothermie). La température monte à 40°C où elle est maintenue pendant 4 h sous agitation efficace.

La masse réactionnelle est ensuite diluée à l'eau et extraite au chloroforme. Les phases organiques réunies sont lavées à l'eau, séchées sur MgSO₄ et concentrées à l'évaporateur rotatif sous pression réduite. Rdt = 80 %.

### Exemple N° 10

### Synthèse de PhCH₂SO₂CF₃

### A partir de PhCH₂SCF₃

12.7 ml d'eau oxygénée à 30 % (124.3 mmol) sont coulés goutte à goutte sur le benzyl trifluorométhyl sulfure (5.96 mg, 31.06 mmol) en solution dans 23 ml d'acide acétique à 99 %. Après 2 h d'agitation à 90°C, la masse réactionnelle est diluée à l'eau et extraite à l'éther (3 x 100 ml). La phase organique est lavée avec : 3 x 75 ml d'eau, 2 x 75 ml de solution saturée de NaHCO3 et 2 x 75 ml d'eau. Puis elle est séchée sur MgSO₄ et concentrée à l'évaporateur rotatif sous pression réduite. Le solide obtenu est recristallisé dans CCl₄ (25 à 30 ml). On obtient 4.604 g de solide blanc dont la structure et la pureté sont contrôlées par RMN. Rdt = 66 % en produit isolé.

RMN ¹H (CDCl₃) :
4,47 ppm (s, 2H)
7,43 ppm (m, 5H, H aromatiques).

RMN ¹⁹F (CDCl₃) :
- 76,91 ppm (s)

### Exemple N° 11

### Synthèse de la 1,2-diphénylethyltriflone

La benzyltriflone (0.673 g, 3 mmol) est mise en solution dans 12 ml d'acétonitrile sec. On introduit ensuite K₂CO₃ (0.485 g, 3.5 mmol) puis le bromure de benzyle (0.365 ml, 3 mmol). La masse réactionnelle est portée à reflux (82°C) pendant 20 h. Elle est ensuite filtrée, diluée à l'eau et extraite à l'éther (2 x 25 ml). Les phases organiques réunies sont lavées à l'eau (2 x 20 ml) et avec une solution saturée de NaCI (1 x 20 ml). Après séchage sur MgSO₄ et concentration à l'évaporateur rotatif sous pression réduite, on obtient une huile jaune. Le produit est recristallisé dans 3 ml d'éther de pétrole. RDT = 68 % à 82 % en produit isolé (0.637 g à 0.768 g de solide blanc) caractérisé par RMN.

| | |
|---|---|
| RMN ¹H (CDCL₃) | 3,39 ppm (dd, 1 H, ²J = 13.6 Hz, ³J = 3.2 Hz, H₂ ou H₃) |
| | 3,77 ppm (dd, 1H, ²J = 13.6 Hz, ³J = 11.6 Hz, H₃ ou H₂) |
| | 4,54 ppm (dd, 1H, ³J = 3.2 Hz, ³J = 11.6 Hz, H₁) |
| | 6,92 à 7,35 ppm (m, 10 H, H aromatiques) |
| | |
| | RMN ¹⁹F (CDCl₃) : |
| | - 73,80 ppm (s) |

### Exemple N° 12

### Synthèse du triflinate par β-élimination sur la 1,2-diphényléthyltriflone

### Base : DBU (diazabicycloundécène)

La triflone (186 mg, 0.592 mmol), la DBU (104 mg, 0.655 mmol) et le 1,4-dioxanne (3.2 ml) sont chauffés à 100°C pendant 67 h. Après refroidissement, la masse réactionnelle est diluée avec 20 ml de dichlorométhane et lavée avec 10 ml d'eau. La phase aqueuse est extraite avec 2 x 20 ml de dichlorométhane. La phase aqueuse est mise de côté.

Les phase organiques réunies sont lavées avec 1 x 15 ml d'eau (que l'on ajoute à la phase aqueuse précédente), 2 x 20 ml d'eau et 1 x 20 ml d'une solution saturée de NaCI. Après séchage sur MgSO₄ et concentration à l'évaporateur rotatif sous pression réduite, on récupère 134 mg d'un solide blanc-jaunâtre analysé par RMN ¹H (CDCl₃). C'est un mélange contenant :
- du trans-stilbène (spectre identique à la référence trouvée dans la littérature (20))
- du produit de départ (spectre identique à celui décrit précédemment)
- des impuretés non identifiées.

La proportion de trans-stilbène par rapport au produit de départ est de 70/30 (% molaires).

La phase aqueuse mise de côté est rendue basique par un ajout de 5.9 ml de solution de soude 0.1N. Elle est ensuite extraite avec 4 x 30 ml de dichlorométhane (extraction de la DBU), neutralisée à l'acide chlorhydrique 0.1 N, extraite avec 4 x 20 ml de toluène (extraction des résidus organiques) et concentrée à l'évaporateur rotatif sous pression réduite. Le dépôt jaune huileux obtenu (116 mg) est caractérisé et dosé par RMN (étalon : CF₃CH₂OH). Rdt = 2 %.
RMN ¹⁹F(H2O) : - 87.2 ppm (s,CF₃SO₂Na)

### Base : NaH

Dans un ballon de 10 ml, on introduit l'hydrure de sodium en suspension à 50 % dans l'huile (29 mg, 0.604 mmol). Le ballon est purgé à l'azote. On coule ensuite 3 ml de THF puis la 1,2-diphényléthyltriflone (188 mg, 0.599 mmol) en solution dans 1 ml de THF. Le milieu est agité à température ambiante sous balayage d'azote. Après 6 h d'agitation, on rajoute de l'hydrure de sodium (10 mg, 0.208 mmol). La réaction est suivie par CPG (les temps de rétention du produit de départ et des produits d'arrivée possibles sont connus grâce à des échantillon authentiques).

### Base : MeOna

760 mg de 1,2-diphényléthyltriflone (2.42 mmol), 408 mg de méthylate de sodium (7.55 mmol) et 10 ml de méthanol anhydre sont agités pendant 24 h à 60°C.
La masse réactionnelle est ensuite concentrée à l'évaporateur rotatif sous pression réduite, diluée dans 45 ml d'un mélange éther/eau (2/1) et décantée. La phase aqueuse est extraite avec 4 x 25 ml de CH₂Cl₂. Les phases organiques rassemblées sont lavées avec 1 x 25 ml d'eau que l'on ajoute à la phase aqueuse précédente, 2 x 40 ml d'eau et 1 x 40 ml de saumure. Après séchage sur MgSO₄ et concentration à l'évaporateur rotatif sous pression réduite, on obtient 0.747 g de solide blanc caractérisé et dosé par RMN¹H (étalon : CH₂Cl₂). C'est un mélange contenant :
- 1.82 mmol de trans-stilbène (Rdt = 75 %)
- 0.58 mmol de produit de départ (soit 24 % de la charge initiale)
Les déplacements δ sont identiques à ceux décrits précédemment.

La phase aqueuse est traitée par une solution d'HCl 6N, jusqu'à ce que son pH soit environ 5, puis concentrée à l'évaporateur rotatif sous pression réduite. On récupère 0.514 g de solide blanc analysé par RMN¹⁹F (étalon : CF₃CH₂OH). Rdt = 72 % en triflinate de sodium (spectre identique à celui décrit précédemment).

### Exemple N° 13

### Synthèse du triflinate de potassium par β-élimination sur le 3-phényl, 3-(trifluorométhanesulfonyl)propionate d'éthyle

Dans un ballon de 25 ml, on charge la benzyltriflone (559 mg, 2.5 mmol), le carbonate de potassium (1043 mg, 7.55 mmol) et 10 ml d'acétonitrile. On coule le bromoacétate d'éthyle (300 µl, 2.65 mmol) en une seule fois. Le ballon est ensuite fermé et chauffé à 80°C sous agitation pendant 16 h.

Après refroidissement, la masse réactionnelle est diluée dans 30 ml d'eau et 60 ml d'Et₂O. Le mélange est décanté. La phase aqueuse est extraite avec 40 ml d'Et₂O. Les phases organiques rassemblées sont lavées avec 4 x 40 ml d'eau. Après séchage sur MgSO4 et concentration à l'évaporateur rotatif sous pression réduite, on obtient 400 mg de liquide jaune. Rdt = 88 % en produit caractérisé par RMN¹H.
Toutes les phases aqueuses sont rassemblées, neutralisées avec une solution d'HCL 0.1N et concentrées à l'évaporateur rotatif sous pression réduite (T = 65°C). L'eau résiduelle est éliminée par entraînement azéotropique au toluène. On obtient ainsi 1.156 g de solide blanc analysé par RMN¹⁹F (étalon : CF₃CH₂OH). Rdt = 68 %. Le triflinate de potassium est extrait avec 8 x 10 ml d'acétate d'éthyle. On isole 390 mg de solide blanc. RDT = 49 % en produit isolé caractérisé par RMN¹⁹ F.

## Revendications

1. Procédé pour la synthèse de dérivés fluorés, **caractérisé par le fait qu'**il comporte une étape d'échange entre le fluor et un halogène plus lourd que le fluor, dans laquelle un substrat comportant au moins un carbone halogénophore d'hybridation sp³ porteur d'au moins deux halogènes ci-après désignés par X et X', dont l'un au moins est un halogène plus lourd que le fluor, lequel carbone halogénophore est relié à un chalcogène au moins, et soumis à l'action d'un réactif comportant au moins un composé défini choisi parmi ceux constitués par l'association d'une base de Bronstedt avec un nombre défini n d'acides fluorhydriques, n étant au moins égal à 3 et au plus à 20, avantageusement, de préférence à 10, et **par le fait que** ledit substrat correspond à la formule :
R-CXX'-Y(O)ᵣ-R₅
• avec R choisi parmi les restes hydrocarbonés, les halogènes, les électro-attracteurs et les hydrocarbyles chalcogényles ;
• avec X représentant le le chlore ;
• avec X' représentant le chlore ;
• avec Y choisi parmi les chalcogènes avantageusement de rang atomique supérieur à l'oxygène, notamment quand R est différent des hydrocarbyles chalcogényles et avec la condition que lorsque Y est oxygène, R est égal à 0 ;
• où r représente 0 ou un entier choisi parmi 1 ou 2 et est avantageusement inférieur à 2 ;
• R₅ représente un radical :
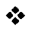 aryle éventuellement substitué, en particulier hétéroaryle ;
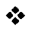 alcoyle dont le carbone d'hybridation sp³ constitue un ensemble moins éléctroattracteur qu'un dichlorophényle;
et **par le fait que** chaque radical R et R₅ comporte au plus 30, de préférence au plus 15 atomes de carbone et/ou d'azote, le nombre total de carbones des molécules substrats ne dépassant pas 50, avantageusement 30.

2. Procédé selon la revendication 1, **caractérisé par le fait que** R est un groupe choisi parmi les groupes électroattracteurs par effet inductif.

3. Procédé selon les revendications 1 et 2, **caractérisé par le fait que** la somme des nombres atomiques dudit ou desdits chalcogènes(s) est au moins égal à 10.

4. Procédé selon les revendications 1 à 3, **caractérisé par le fait que** ledit groupe R est choisi parmi les groupes électroattracteurs est un halogène.

5. Procédé selon les revendications 1 à 4, **caractérisé par le fait que** ledit carbone halogénophore porte au moins deux halogènes de nombre atomique supérieur à celui du fluor.

6. Procédé selon les revendications 1 à 5, **caractérisé par le fait que** ledit carbone halogénophore porte trois halogènes choisis parmi le chlore et le fluor.

7. Procédé selon les revendications 1 à 6, **caractérisé par le fait que** ledit chalcogène Y est divalent.

8. Procédé selon les revendications 1 à 7, **caractérisé par le fait que** ledit chalcogène Y est lié par sa seconde liaison à un atome électrodonneur par effet inductif ou mésomère avec la condition qu'il ne soit pas oxygène lorsque le chalcogène lié au carbone halogénophore l'est.

9. Procédé selon la revendication 8, **caractérisé par le fait que** ledit atome électrodonneur est un carbone appartenant à un radical alcoyle ou à un radical arylique riche en électron.

10. Procédé selon la revendication 9, **caractérisé par le fait que** ledit alcoyle est un radical aralcoyle, de préférence choisi parmi les radicaux benzyles.

11. Procédé selon la revendication 9, **caractérisé par le fait que** ledit radical arylique riche en électron est un radical hétérocyclique à cinq chaînons.

12. Procédé selon les revendications 1 à 11, **caractérisé par le fait que** ledit chalcogène est du soufre et qu'il comporte une étape ultérieure d'oxydation dudit atome de soufre.

13. Procédé selon la revendication 12 , **caractérisé par le fait que** ladite étape d'oxydation est réalisée pour obtenir ledit atome de soufre sous forme d'une sulfone.

14. Procédé selon la revendication 13, **caractérisé par le fait qu'**il comporte une étape ultérieure d'hydrolyse avantageusement en milieu alcalin pour donner un sel ou l'acide sulfinique ou sulfonique correspondant.

15. Procédé selon la revendication 12 , **caractérisé par le fait que** ladite oxydation est réalisée pour obtenir ledit atome de soufre sous la forme d'un sulfoxyde, d'un sulfènate ou sous une forme présentant le même état d'oxydation que celui d'un sulfoxyde, ou que celui d'un un sulfénate.

16. Procédé selon la revendication 15, **caractérisé par le fait que** la dite oxydation est menée dans un milieu protique avantageusement en présence d'un acide carboxylique, par addition d'hydroperoxyde (de préférence d'eau oxygénée) et maintien à une température de 20°C à 80°C, pendant une durée pouvant varier d'environ 1 h à deux jours.

17. Procédé selon les revendications 15 et 16, **caractérisé par le fait qu'**il comporte une étape ultérieure de d'halogénation, de préférence de chloration, modérée pour obtenir un halogénure de sulfinyle.

18. Procédé selon la revendication 17, **caractérisé par le fait que** la dite halogénation est menée en soumettant ledit sulfénate, avantageusement dilué dans un solvant peu polaire (c'est-à-dire incapable de dissoudre plus de 5 % en masse d'eau), essentiellement anhydre (c'est-à-dire dont l'eau est présent en quantité d'au plus 1/3 en mole du substrat, avantageusement d'au plus 1/5, de préférence d'au plus 1/10) et peu sensible au chlore, à l'action du chlore au moins en quantité stoechiométrique, à une température au plus égale à 100°C, avantageusement entre 0°C et 50°C.

19. Procédé selon les revendications 1 à 18, **caractérisé par le fait que** ladite base de Bronstedt est choisie parmi les pnictines et les hétérocycles aromatique dans lesquels ledit hétéroatome est choisi parmi les atomes de la colonne V (colonne de l'azote).

20. Procédé selon les revendications 1 à 19, **caractérisé par le fait que** la teneur en mercure et en argent soit telle que le rapport molaire entre le métal et le substrat dont les atomes d'halogène sont à échanger, se situe à une valeur au plus égale à 0,1.

21. utilisation comme réactif d'échange chlore-fluor d'un composé défini choisi parmi ceux constitués par l'association d'une base de Bronstedt avec un nombre défini n d'acide fluorhydrique, n étant au moins égal à 3 et au plus à 5, ladite base de Bronstedt étant choisie parmi les amines primaires, secondaires et de préférence tertiaires.

## Claims

1. Process for synthesizing fluoro derivatives, **characterized in that** it includes a step of exchange between fluorine and a halogen heavier than fluorine, in which a substrate comprising at least one halophoric carbon of sp³ hybridization bearing at least two halogens referred to hereinbelow as X and X', at least one of which is a halogen heavier than fluorine, which halophoric carbon is linked to at least one chalcogen, and subjected to the action of a reagent comprising at least one defined compound chosen from those consisting of the combination of a Bronstedt base with a defined number n of hydrofluoric acids, n being at least equal to 3 and advantageously not more than 20 and preferably 10, and **in that** the said substrate corresponds to the formula:
R-CXX'-Y(O)ᵣ-R₅
• with R chosen from hydrocarbon-based residues, halogens, electron-withdrawing groups and chalcogenyl hydrocarbyles;
• with X representing chlorine;
• with X' representing chlorine;
• with Y chosen from chalcogens advantageously of an atomic row higher than oxygen, especially when R is other than chalcogenyl hydrocarbyles and with the condition that, when Y is oxygen, R is equal to 0;
• in which r represents 0 or an integer chosen from 1 and 2 and is advantageously less than 2 ;
• R₅ represents a radical:
* optionally substituted aryl, in particular heteroaryl;
* alkyl in which the carbon of sp³ hybridization is a unit that is less electron-withdrawing than a dichlorophenyl;
and **in that** each radical R and R₅ contains not more than 30 and preferably not more than 15 carbon and/or nitrogen atoms, the total number of carbons in the substrate molecules not exceeding 50 and advantageously 30.

2. Process according to Claim 1, **characterized in that** R is a group chosen from groups which are electron-withdrawing by an inductive effect.

3. Process according to Claims 1 and 2, **characterized in that** the sum of the atomic numbers of the said chalcogen or chalcogens is at least equal to 10.

4. Process according to Claims 1 to 3, **characterized in that** the said group R chosen from electron-withdrawing groups is a halogen.

5. Process according to Claims 1 to 4, **characterized in that** the said halophoric carbon bears at least two halogens of atomic number higher than that of fluorine.

6. Process according to Claims 1 to 5, **characterized in that** the said halophoric carbon bears three halogens chosen from chlorine and fluorine.

7. Process according to Claims 1 to 6, **characterized in that** the said chalcogen Y is divalent.

8. Process according to Claims 1 to 7, **characterized in that** the said chalcogen Y is linked via its second bond to an atom which is electron-donating by an inductive or mesomeric effect, with the condition that it is not oxygen when the chalcogen linked to the halophoric carbon is an oxygen.

9. Process according to Claim 8, **characterized in that** the said electron-donating atom is a carbon belonging to an alkyl radical or to an electron-rich aryl radical.

10. Process according to Claim 9, **characterized in that** the said alkyl is an aralkyl radical, preferably chosen from benzyl radicals.

11. Process according to Claim 9, **characterized in that** the said electron-rich aryl radical is a five-membered heterocyclic radical.

12. Process according to Claims 1 to 11, **characterized in that** the said chalcogen is sulphur and **in that** it includes a subsequent step of oxidation of the said sulphur atom.

13. Process according to Claim 12, **characterized in that** the said oxidation step is performed in order to obtain the said sulphur atom in the form of a sulphone.

14. Process according to Claim 13, **characterized in that** it includes a subsequent step of hydrolysis, advantageously in alkaline medium, to give a corresponding sulphinic or sulphonic acid salt.

15. Process according to Claim 12, **characterized in that** the said oxidation is performed in order to obtain the said sulphur atom in the form of a sulphoxide, of a sulphenate or in a form that has the same oxidation state as that of a sulphoxide, or as that of a sulphenate.

16. Process according to Claim 15, **characterized in that** the said oxidation is carried out in a protic medium, advantageously in the presence of a carboxylic acid, by addition of hydroperoxide (preferably aqueous hydrogen peroxide solution) and is maintained at a temperature of 20°C to 80°C for a period which may range from about 1 h to two days.

17. Process according to Claims 15 and 16, **characterized in that** it includes a subsequent step of halogenation, preferably of chlorination, which is moderated in order to obtain a sulphinyl halide.

18. Process according to Claim 17, **characterized in that** the said halogenation is carried out by subjecting the said sulphenate, advantageously diluted in a sparingly polar solvent (i.e. a solvent, that is incapable of dissolving more than 5% by mass of water), which is essentially anhydrous (i.e. a solvent in which water is present in an amount of not more than 1/3 mol, advantageously not more than 1/5 mol and preferably not more than 1/10 mol of the substrate) and which shows little sensitivity to chlorine, to the action of chlorine in an at least stoichiometric amount, at a temperature at most equal to 100°C, advantageously between 0°C and 50°C.

19. Process according to Claims 1 to 18, **characterized in that** the said Bronstedt base is chosen from pnictines and aromatic heterocycles in which the said hetero atom is chosen from the atoms of column V (the nitrogen column).

20. Process according to Claims 1 to 19, **characterized in that** the content of mercury and of silver are such that the molar ratio between the metal and the substrate of which the halogen atoms are to be exchanged has a value of not more than 0.1.

21. Use, as a chlorine-fluorine exchange reagent, of a defined compound chosen from those consisting of the combination of a Bronstedt base with a defined number n of hydrofluoric acid, n being at least equal to 3 and not more than 5, the said Bronstedt base being chosen from primary, secondary and, preferably, tertiary amines.

## Patentansprüche

1. Verfahren zur Synthese von fluorierten Derivaten, **dadurch gekennzeichnet, daß** es eine Stufe des Austausches zwischen dem Fluor und einem schwereren Halogen als dem Fluor umfaßt, in der ein Substrat, das mindestens einen halogenophoren Kohlenstoff der Hybridisierung sp³ umfaßt, der seinerseits mindestens zwei nachfolgend mit X und X' bezeichnete Halogene trägt, von denen mindestens eines ein schwereres Halogen als Fluor ist, und wobei der halogenophore Kohlenstoff mit mindestens einem Chalkogen verbunden ist, der Einwirkung eines Reaktanden unterzogen wird, der mindestens eine definierte Verbindung umfaßt, ausgewählt unter denen, die durch Assoziation einer Bronstedt-Base mit einer definierten Anzahl n von Fluorwasserstoffsäuren gebildet werden, worin n mindestens gleich 3 und vorteilhafterweise höchstens 20, vorzugsweise 10 ist, und dadurch, daß das genannte Substrat der Formel
R-CXX'-Y(O)ᵣ-R₅
entspricht,
· mit R ausgewählt unter den Kohlenwasserstoff-Resten, den Halogenen, den Elektroattraktoren und den chalkogenylierten Hydrocarbylen;
· mit X, das Chlor darstellt;
· mit X', das Chlor darstellt;
· mit Y, ausgewählt unter den Chalkogenen mit vorteilhafterweise einem Atomrang von höher als dem des Sauerstoffs, insbesondere wenn R von chalkogenylierten Hydrocarbylen verschieden ist, und mit der Bedingung, daß in dem Fall, wo Y Sauerstoff bedeutet, R gleich 0 ist;
· worin r 0 oder ein Ganzes darstellt, ausgewählt unter 1 oder 2 und vorteilhafterweise unterhalb von 2;
· R₅ einen Rest darstellt:
* Aryl, gegebenenfalls substituiert, insbesondere Heteroaryl;
* Alkyl, bei dem der Hybridisierungskohlenstoff sp³ eine weniger elektroattraktive Einheit als ein Dichlorphenyl bildet;
und dadurch, daß jeder Rest R und R₅ höchstens 30, vorzugsweise höchstens 15 Atome von Kohlenstoff und/oder Stickstoff umfaßt, wobei die Gesamtanzahl der Substratmoleküle an Kohlenstoffen 50, vorteilhafterweise 30, nicht übersteigt.

2. Verfahren nach Anspruch 1, **dadurch gekennzeichnet, daß** R eine Gruppe darstellt, ausgewählt unter den elektroattraktiven Gruppen durch induktive Wirkung.

3. Verfahren nach Anspruch 1 und 2, **dadurch gekennzeichnet, daß** die Summe der Atomnummern des oder der genannten Chalkogens(e) mindestens gleich 10 beträgt.

4. Verfahren nach Anspruch 1 bis 3, **dadurch gekennzeichnet, daß** die genannte Gruppe R, die unter den elektroattraktiven Gruppen ausgewählt wird, ein Halogen ist.

5. Verfahren nach Anspruch 1 bis 4, **dadurch gekennzeichnet, daß** der genannte halogenophore Kohlenstoff mindestens zwei Halogene mit einer Atomnummer von über der des Fluors trägt.

6. Verfahren nach Anspruch 1 bis 5, **dadurch gekennzeichnet, daß** der genannte halogenophore Kohlenstoff drei Halogene trägt, ausgewählt unter Chlor und Fluor.

7. Verfahren nach Anspruch 1 bis 6, **dadurch gekennzeichnet, daß** das genannte Chalkogen Y divalent ist.

8. Verfahren nach Anspruch 1 bis 7, **dadurch gekennzeichnet, daß** das genannte Chalkogen Y über seine zweite Bindung durch induktive oder mesomere Wirkung mit einem Elektrodonatoratom verbunden ist, mit der Bedingung, daß es keinen Sauerstoff darstellt, wenn das Chalkogen an den halogenophoren Kohlenstoff gebunden ist.

9. Verfahren nach Anspruch 8, **dadurch gekennzeichnet, daß** das genannte Elektrodonatoratom ein Kohlenstoff ist, der von einem Rest Alkyl oder von einem an Elektronen reichen Rest Aryl stammt.

10. Verfahren nach Anspruch 9, **dadurch gekennzeichnet, daß** das genannte Alkyl ein Rest Aralkyl ist, vorzugsweise ausgewählt unter den Resten Benzyl.

11. Verfahren nach Anspruch 9, **dadurch gekennzeichnet, daß** der an Elektronen reiche Rest Aryl ein heterocyclischer Rest mit fünf Ringgliedern ist.

12. Verfahren nach Anspruch 1 bis 11, **dadurch gekennzeichnet, daß** das genannte Chalkogen Schwefel ist und daß es eine spätere Stufe der Oxidation des genannten Schwefelatoms umfaßt.

13. Verfahren nach Anspruch 12, **dadurch gekennzeichnet, daß** die genannte Stufe der Oxidation realisiert wird, um das genannte Schwefelatom in Form eines Sulfons zu erhalten.

14. Verfahren nach Anspruch 13, **dadurch gekennzeichnet, daß** es eine spätere Stufe der Hydrolyse umfaßt, vorteilhafterweise im alkalischen Medium, um ein Salz oder die entsprechende Sulfinsäure oder Sulfonsäure zu ergeben.

15. Verfahren nach Anspruch 12, **dadurch gekennzeichnet, daß** die genannte Oxidation realisiert wird, um das genannte Schwefelatom in Form eines Sulfoxides, eines Sulfenates oder in einer Form zu erhalten, die den gleichen Oxidationszustand aufweist wie der eines Sulfoxides oder wie der eines Sulfenates.

16. Verfahren nach Anspruch 15, **dadurch gekennzeichnet, daß** die genannte Oxidation in einem protischen Medium, vorteilhafterweise in Anwesenheit einer Carbonsäure, mittels Addition von Hydroperoxid (vorzugsweise Wasserstoffperoxid) unter Aufrechterhalten einer Temperatur von 20 °C bis 80 °C und während einer Dauer durchgeführt wird, die von etwa 1 Stunde bis zu zwei Tagen variieren kann.

17. Verfahren nach Anspruch 15 und 16, **dadurch gekennzeichnet, daß** es eine spätere Stufe der gemäßigten Halogenierung, vorzugsweise Chlorierung umfaßt, um ein Sulfinylhalogenid zu erhalten.

18. Verfahren nach Anspruch 17, **dadurch gekennzeichnet, daß** die Halogenierung durchgeführt wird, indem man das genannte Sulfenat, vorteilhafterweise verdünnt in einem wenig polaren (das heißt unfähig, mehr als 5 Masse-% Wasser zu lösen) und im wesentlichen wasserfreien (das heißt, bei dem das Wasser in einer Menge von höchstens 1/3 in Mol des Substrates, vorteilhafterweise höchstens 1/5, vorzugsweise höchstens 1/10 anwesend ist) und gegenüber Chlor wenig empfindlichen Lösungsmittel der Einwirkung von Chlor in mindestens stöchiometrischer Menge sowie bei einer Temperatur von höchstens gleich 100 °C, vorteilhafterweise zwischen 0 °C und 50 °C unterzieht.

19. Verfahren nach einem der Ansprüche 1 bis 18, **dadurch gekennzeichnet, daß** die genannte Bronstedt-Base unter den Pnictinen und den aromatischen Heterocyclen ausgewählt wird, bei denen das genannte Heteroatom unter den Atomen der Reihe V (Stickstoffreihe) ausgewählt wird.

20. Verfahren nach Anspruch 1 bis 19, **dadurch gekennzeichnet, daß** der Gehalt an Quecksilber und an Silber derart sein soll, daß das molare Verhältnis zwischen dem Metall und dem Substrat, bei dem die Halogenatome auszutauschen sind, bei einem Wert von höchstens gleich 0,1 liegt.

21. Verwendung einer definierten Verbindung als Reagens zum Austausch Chlor-Fluor, ausgewählt unter denen, die durch Assoziation einer Bronstedt-Base mit einer definierten Anzahl n von Fluorwasserstoffsäure gebildet werden, wobei n mindestens gleich 3 und höchstens 5 beträgt und die genannte Bronstedt-Base unter den primären, sekundären und vorzugsweise tertiären Aminen ausgewählt wird.
